# EUROPEAN PATENT APPLICATION

(11) **EP 2 039 776 A2**
(43) Date of publication of application: **25.03.2009**
(21) Application number: 08105201.1
(22) Date of filing: 02.09.2008
(51) Int. Cl.: C12P 5/02

(54) **Method of processing slops**

(30) Priority: 20.09.2007 CZ 20070657
(71) Applicant: EcoFuel Labs LLC, Vancouver WA 98664-2618 (US)
(72) Inventor:
(74) Representative: Rezac, Petr

(57) **Abstract**

A method of processing slops including production of biogas characterized in that slops are exposed to anaerobic fermentation in a anaerobic fermenter under generation of biogas and at least a portion of the biogas containing methane and carbon dioxide is fed into a first photo-bioreactor, where the carbon dioxide or a part thereof is consumed during a photosynthesis process producing micro-algae and methane and the residual carbon dioxide are hen withdrawn for further processing.

## Description

### Technical Field

The invention relates to a method of processing slops including production of biogas. Slops originate as a waste mash remaining after distillation of bioalcohol from various natural raw materials, such as beet and cane molasses including fermented products of hydrolysis of starch materials, more specifically potato and maize or lignocellulosic waste, etc...

### Background Art

It is widely known that in order to protect environment liquid fuels of fossil origin such as petrol and diesel oil are partially substituted by products of plant origin for example so called bioalcohol and biodiesel. The quantity of slops resulting from the bioalcohol production may reach a ratio 20:1 in proportion to the ethanol distilled. Although such slops are not classified as a hazardous waste, they may significantly affect environment. It is also known that one distillery producing for example in average 100 tons slops affects environment to the extent, which is equivalent to a town of 50,000 inhabitants. Therefore, any disposal of slops is urgently needed. The following table shows certain characteristic values of various slops types resulting from various basic raw materials after their fermentation:

Raw material Ethanol yield l/kg Slops yield l/kg BSK₅ CHSK gr/l

Beet molasses 0,2 2,2 38 65

Cane molasses 0,32 3,8 35 60

Corn 0,38 6.3 37 56

Wood 0,25 20,4 13 25

The amounts of biogenic elements in slops are rather different and depend on the nature of the initial raw materials. The total amount of nitrogen is ranging from 600 to 4000 mgr/l phosphor, lime - from 30 to 200 mgr/l, potassium - from 40 to 10 000 mgr/l, total sulphur amount - expressed as a sulphate anion - from 600 to 4000 mgr/l, whereby the acidity is in a range from pH 4 to pH 5.5. Accordingly, slops represent an unpleasant waste on the one hand, which waste may be on the other hand advantageously exploited as a valuable raw material with respect to their content of organic and inorganic substances.

Until now, slops were used mostly as an additional fertilizer or were immobilized on various matrixes to ease their liquidation. In view of the expected growth of bioalcohol production in the near future and consequently the increase of the slops production, it is necessary to find a method of reducing their volume and in particular, their useful exploitation.

According to the state of the art references, anaerobic fermentation of slops for producing biogas assigned to be used as a source of energy in a cogeneration unit is regarded as a most suitable method of exploitation. Nevertheless, slops are not exploited in complex i.e. they are exploited for energetic purposes only and not for their further handling, in order to obtain desired products. The resulting situation is that for example in production of 100 ton/day slops, the simple return of the invested capital is 7.8 years without state subsidies and 5.6 years with state subsidies, what represents economically non-perspective payback periods (data: Institute of Agricultural Technology of Prague). Moreover, the costs of clarification of effluent water from anaerobic fermenter are not included in this calculation although they may be very high.

It is also known that various kinds of algae or microalgae are able to consume various specific amounts of carbon dioxide, usually specified as kg CO₂/kg dry algae. Until now, an algae suspension in a photo-bioreactor was mostly doped by carbon dioxide or only recently attempts were made to dope the algae suspension by flue gases, produced for example in thermal power stations or incineration plants. It may be easily calculated that for the retention of carbon oxides escaping from a current thermal power plants the active surfaces of bioreactors for retention of carbon dioxide from flue gases would be enormous in consideration of the fact that an average amount of carbon dioxide consumed for the growth of biomass of algae Chlorella is about 4.4 kg CO₂/kg algae at the current bioreactors outputs of 10 - 25 gr/m² photo-reactor effective area per day with the open photo-bioreactors such as open-air ponds and/or 0.5 -. 2.2 gr/l photo-reactor effective area per day with the closed tube reactors. On the other hand, fermenters for production of biogas offers always an appropriate amount of exploitable carbon dioxide, which may be consumed by algae cultivated photo-bioreactors of standard size.

### Disclosure of Invention

A primary object of the invention is to provide comprehensive exploitation of slops in order to produce energy and valuable raw materials. Another object of the invention is to perform such production on a large industrial scale and to speed up significantly the return of capital invested in slops disposal in comparison with a simple anaerobic process relying on mere biogas production.

The said objects of the invention are achieved by a method of processing slops including biogas production according to which slops are exposed to anaerobic fermentation in an anaerobic fermenter under generation of biogas and at least a portion of the biogas containing methane and carbon dioxide is fed into a first photo-bioreactor, where the carbon dioxide or a part thereof is consumed during a photosynthesis process producing microalgae and methane and the residual carbon dioxide are then withdrawn for further processing.

According to one aspect of the invention methane and residual carbon dioxide are withdrawn from the photo-bioreactor and fed into a cogeneration unit to produce electric current and utilizable heat and flue gases from the cogeneration unit containing carbon dioxide may be fed to a second photo-bioreactor to produce another type of micro-algae.

According to another aspect of the invention the waste water resulting from anaerobic fermentation of slops in the anaerobic fermenter is supplied together with a portion of slops, calc and plant remains, such as straw, bran, compost, grout obtained from a rape seed pressing oil production to a mixer to obtain a mixture, which is further carried into a gravity settler in which a solid, partially aqueous sediment is separated from the aqueous phase, whereby the sediment is periodically or continuously removed from the settler and the aqueous phase is brought in a cloth filter or a centrifuge to separate solid particles from the aqueous phase, which is subsequently fed into an evaporator and a gas phase from the evaporator together with a portion of biogas produced in anaerobic fermentation is supplied to an absorption column sprinkled by water, from which the non-absorbed gases are fed to the first bio-reactor and/or the cogeneration unit and the aqueous solution from the absorption column is fed to the second bio-reactor.

According to still another aspect of the invention, a portion of slops is added to photo-bioreactors and at least one photo-bioreactor may be supplied with carbon dioxide from fermentation vessels used in production of bioalcohol.

Advantageously, the first photo-reactor is operated as a closed photo-reactor and the second photo-reactor is operated as an open photo-reactor.

In summary, the method of processing slops according to the invention includes production of energy and micro-algae, wherein after the bioalcohol distillation in an anaerobic fermenter a biogas is produced containing almost exclusively methane and carbon dioxide. Carbon dioxide is consequently used in a photo-reactor in a photosynthetic production of microalgae, whereby methane is exploited in a cogeneration unit for production of useful electric and thermal energy. A part of slops is simultaneously used as a liquid substrate for producing microalgae for their content of additional nutrients, which are rich in nitrogen and phosphorus as elements necessary for microalgae production. Accordingly, this method represents a complete exploitation of slops as a waste mash after distillation of bioalcohol for production of electric and thermal energy and microalgae. Thus, waste in the form of slops is a raw material for production of electric and thermal energy and simultaneously for obtaining new products such as microalgae, which are further usable as a raw material highly in demand in the consumer industry. An efficient reasonable exploitation of slops thus ranks among environmental protection technologies since, according to the method presented, the waste is

exploited as a raw material in a process of production biogas, where carbon dioxide as a greenhouse gas, which is normally discharged into atmosphere, is completely disposed of without affecting the atmosphere. The method according to the invention further differs from other methods relying on production of biogas for energetic purposes only in that it uses slops not only in an conventional process of energy production but in an original combination of both processes, wherein carbon dioxide from biogas and nitrogen from slops is used for production of microalgae. Accordingly, the energetic balance of the process of biogas production is significantly improved.

### Brief Description of Figures in the Drawings

### Best Mode for Carrying Out the Invention

### Example 1

Slops 30 collected in a silo 1 are withdrawn from the silo 1 to an anaerobic fermenter 2. Here is the portion of organic substances contained in slops 30 converted into biogas the main components of which are methane and carbon dioxide. Biogas leaves the fermenter 2 and is distributed into three manifolds. The manifold 3 opens directly in a closed photo-bioreactor 6 comprising a micro-algae suspension and the carbon dioxide from biogas is there used as a component necessary for the photosynthesis of biomass in algae. In the bioreactor 6, most of the carbon dioxide, i.e. more than 85 per cent thereof, is absorbed. The unabsorbed methane together with a small amount of unabsorbed carbon dioxide is fed to a cogeneration unit 7 where electric current 8 and utilizable heat 9 is produced. The heat is used for heating fermenter 2 and photo-bioreactors 6 and 10. The cogeneration unit 7 is a commercially available facility. The electric current is sold to the public network suppliers and the heat is utilized for heating the fermenter 2 and, as the case may be, for heating a suspension in the photo-bioreactors 6 and 10 or for heating a boiler as a part of a separation unit 13. The flue gases from the cogeneration unit 7 are supplied to a second bioreactor 10, which may operate in an open or closed mode. The photo-bioreactors 6 and 10 are illuminated by both the daily light and the artificial light. The photo-bioreactors 6 and 10 are facilities for producing algae and their designs are known as well. The second manifold 4 brings biogas in the cogeneration unit 7 to produce electric energy and usable thermal energy. Waste water 25 from the fermenter 2 is fed to the mixer 11, into which solid lime from a dozer 23 and plant residues such as straw and crushed straw, bran and compost are dosed in stoichiometric amounts, and in particular, completed by a portion of slops from a silo 1 dosed over a valve 22. In the mixer 11 pH>8 is maintained.. From the mixer 11 the suspension together with waste water is pumped into a settler and/or centrifuge 12, where solid particles are separated and waste water continue to enter after passing through a filter 24 a thermal separator - evaporator 13. The solid waste portion from the settler 12 with a low humidity content i.e. about 15 per cent, is withdrawn as a fertilizer for agricultural purposes. Two streams leave the evaporator 13: a waste water stream 14a and a gas phase - gases 14b. A substantial component of gases 14b is ammonia. The gases 14b leaving the evaporator 15 are absorbed in an absorption column 15 together with the biogas supplied to the column 15 from the manifold 5. The absorption column 15 is sprinkled by water. Water 16 leaving the absorption column 15 enriched by dissolved carbon dioxide and ammonium is fed to the photo-bioreactor 10. The non-absorbed methane leaves the absorption column 15 through pipeline 26 and enters the cogeneration unit 7 where it is oxidized to carbon dioxide, which is then, in the form of flue gases, supplied to the photo-bioreactor 10.

Both the photo-bioreactors 6, 10, are operated in the mode of biomass recycling and after the biomass collection, the aqueous solutions 18 and 19 are discharged to a water stream. All three manifolds 3, 4, 5 are operated independently or the individual manifolds are operated under a joint control. The water streams entering both the photo-bioreactors 6 and 10 recirculate therein and then, following the separation of algae, are discharged to a water stream as streams 18 and 19. The separated algae 27 and 28 from the photo-bioreactors 6, 10, are further handled as streams and contribute to a positive economic balance of this complex unit. According to a preset procedure, in addition to nutrients on the basis of nitrogen, phosphor and silicon salts, a part of slops is added to the photo-bioreactors 6, 10 as well. Thus, both the problem of handling alcohol distillery waste products and the problem of treatment of waste water leaving the biogas producing fermenter 2 are simultaneously resolved, whereby nitrogen from waste water is used as an additional nutrient for algae 28. The solid plant components 23, such as crushed straw, are dosed together with lime into the mixer 11, where they contribute to the absorption of colloidal organic and inorganic substances from waste water and thus facilitate the sedimentation in the settler 12.

The method according to the invention makes use of two photo-bioreactors 6, 10, what, among others, enables the production of two various types of algae.

In the photo-bioreactor 6, which is advantageously operated as a closed reactor, algae assigned either to direct use, for example as components of food ads or are utilized as a basis for extraction of other valuable substances, such as omega-unsaturated acids, pigments etc., i.e. products which come into direct contact with human beings or animals. The algae suspensions in the photo-bioreactor 6 are saturated by biogas and utilize thus carbon dioxide, which is free from hazardous admixtures, which may otherwise originate for example from burning off biogas and which may be toxic or harmful as current flue gases, which are also used as a source of carbon dioxide.

In the photo-bioreactor 10, algae may be cultivated for production of valuable products, if they are saturated by water withdrawn form the absorber 15. Such water is free from components, which are otherwise produced in burning off biogas. On the other hand, algae assigned to production of oils for their further processing resulting in biofuels may utilize flue gases and carbon dioxide withdrawn from the cogeneration unit 7 as well. The use of water saturated by ammonium and carbonate ions as an algae nutrient represents an original method, which partially replaces the dosing of expensive, clean gaseous carbon dioxide. The photo-bioreactors 6 and 10 may also be supplied directly with the carbon dioxide from a carbon dioxide reservoir, which is produced in the fermentation process in production of bioalcohol 29 and which is fed therein by the pipeline 20.

As to the energy production, all three variants arising from the distribution of the biogas collected in the fermenter 2 into three manifolds 3, 4, 5 are almost equal. Nevertheless, the combination including the biogas distribution into three manifolds enables a simultaneous use of biogas:

- for production of algae 27 only, if a highly sophisticated treatment of waste water from the fermenter 2 is not required, i.e. by distribution of biogas into manifold 3 or manifold 4 only, or simultaneously or alternatively into manifold 3 and 4;

- for production of algae 28 and for simultaneous handling of waste water from fermenter 1 by distributing biogas into the manifold 5 and simultaneously into the manifold 3 and/or manifold 4 or the both manifolds 3 and 4 according to a preset algorithm.

### Example 2

An experimental fermenter for biogas production was fed by a type of mixed slops resulting from alternative processing of corn (bailey) and sugar beet molasses. The content of organic substances in an average sample of such slops was equal to 52 g CHSK/lₛₗₒₚₛ. The fermenter loading was 5 gr CHSK/l_{fermenter}/day. The fermenter useful volume was 50 I. The fermenter is a cylindrical vessel made of stainless steal provided with a jacket heated by circulating water. The temperature of the charge was maintained at 40 °C. The fermenter inoculum was obtained in Prague waste water clarification plant and was used at this temperature in the previous research programs. The fermenter was provided with a paddle wheel stirrer situated near its bottom section (four paddles, d/D = 0.25 where d is the paddle diameter and D is the inner diameter of the cylindrical vessel). The rotation speed was 12 rpm. Slops were dosed into the fermenter together with usual nutrients 2 times per 24 hours. The fermenter was operated in the continuously mode so that before dosing slops an equivalent of waste water was discharged from the fermenter and further handled. The acidity was maintained by automatic dosing of sodium hydroxide to keep it at pH 7. The fermenter was placed on a weighing machine and the constant retention of liquid in the fermenter was checked by weighing. The gas generated in the fermenter was lead into the biogas reservoir through a receptacle provided with a filling comprising Fe²⁺ for trapping sulphane H₂S traces. The average retention time in the fermenter was t = 50/4.8077 = 10.4 days while the dosing speed was 4.808 lₛₗₒₚₛ /day (dosed by 2.404 l, two times a day). The methane yield was high, in average about 0.18 lₘₑₜₕₐₙ /gr COD. In average, the methane production amounted 250 x 0.18 = 45 lₘₑₜₕₐₙₑ /day. The average methane concentration in a stabilized process, which was established after 38 days following the start of charging slops into fermenter was 61 per cent by volume methane and 39 per cent by volume carbon dioxide. The quantity of produced CO₂ was about 28.8 l_{CO2} /day. The biogas flow rate was measured by a gas meter enabling to measure the flow rate at a level of 0.05_{biogas} /minute.

The gas volumes were established under normal conditions 20°C, 0.1 kPa). The biogas from the reservoir was lead to a horizontal tube photo-bioreactor, illuminated by fluorescent lamps with 1.24 W/m²_{fluorescent lamp} intensity. The photo-bioreactor was a transparent plastic tube of 48 mm inner diameter and 1000 mm in length with a static mixer placed inside. Inoculum Chlorella vg. was obtained from the collections of Prague Microbiological Institute of the Czech Academy of Science, Branch Trebon. The algae suspension in the BG 11 medium circulated by means of circulation pump with an output of 0.3 l/sec so that the linear speed of the suspension in the tube (in relation to the tube cross section area) was 0.166 m/sec. The tube was not completely filled with suspension so that in the upper part of the horizontal tube a small gas space was maintained. The tube volume was 1.8086 x 10⁻³ m⁻³, the liquid volume was estimated at about 1.7 litres. The photo-bioreactor was operated in a charge process with a continuous flow of biogas, the suspension was recycled over the photo-bioreactor. The speed of biomass increase was in average 2 g _{biomass}/ l_{liquid}/ day, i.e. the calculated consummation of carbon dioxide in the photo-bioreactor was 8.8 gr/day, since the average amount of carbon dioxide produced by this reactor under the above conditions was 4.4 I CO₂ /gr_{biomass}. The mass flow of carbon dioxide entering the photo-bioreactor was 56.6 g CO₂/day (under normal conditions) in case of 28.8 l CO₂/day. The amount of carbon dioxide produced would satisfy the needs of 6 tubes disposed in parallel and similar to that of the photo-bioreactor. In the described experiment, the gas leaving the photo-bioreactor was discharged into atmosphere. The retention time of algae in the photo-bioreactor was elected to be the same as a middle retention time in the fermenter (10 days), for which time the algae concentrations reached in average 25 g _{biomass dry substance}/l_{liquid}. In total, 255 gr of dry weight biomass were cultivated in repeated charges and for use in further experiments.

### Industrial Application

This invention may be utilized in distilleries producing slops, as an environment affecting waste. The waste slops are used for production of electric energy supplied to a public network while the carbon dioxide produced from such waste and contained in the biogas is simultaneously used for cultivation of algae in photo-bioreactors installed together with a biogas producing fermenter. The produced algae are a valuable raw materials for production of a number of products on demand in the field of food and pharmaceutical industry, cosmetics, as a component of food ads, source of unsaturated omega fatty acids and other chemical products (such as pigments) or for production of liquid bio-fuels.

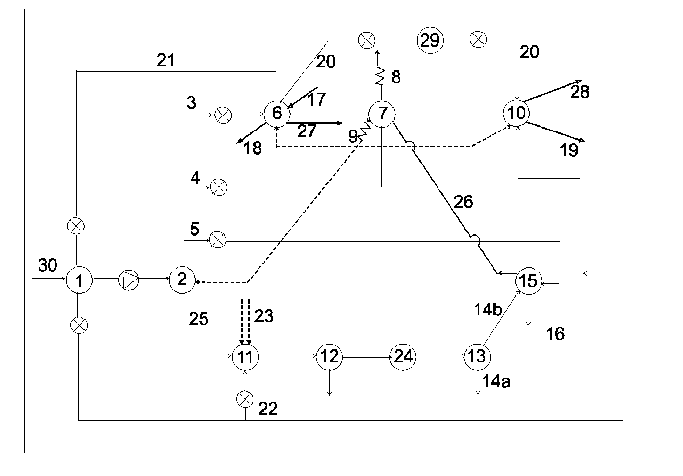

## Claims

1. A method of processing slops including production of biogas **characterized in that** slops are exposed to anaerobic fermentation in an anaerobic fermenter under generation of biogas and at least a portion of the biogas containing methane and carbon dioxide is fed into a first photo-bioreactor, where the carbon dioxide or a part thereof is consumed during a photosynthesis process producing micro-algae and methane and the residual carbon dioxide are then withdrawn for further processing.

2. The method of processing slops according to claim 1, wherein methane and residual carbon dioxide are withdrawn from the photo-bioreactor and fed into a cogeneration unit to produce electric current and utilizable heat.

3. The method of processing slops according to claim 2 wherein flue gases from the cogeneration unit containing carbon dioxide are fed to a second photo-bioreactor to produce another type of micro-algae.

4. The method of processing slops according to any of claim 1 to 3 wherein the waste water resulting from anaerobic fermentation of slops in the anaerobic fermenter, is supplied together with a portion of slops, calc and plant remains, such as straw, bran, compost, grout obtained in a rape seed pressing oil production, to a mixer to obtain a mixture, which is further carried into a gravity settler in which a solid, partially aqueous sediment is separated from the aqueous phase, whereby the sediment is periodically or continuously removed from the settler and the aqueous phase is brought in a cloth filter or a centrifuge to separate solid particles from the aqueous phase, which is subsequently fed into an evaporator and a gas phase from the evaporator together with a portion of biogas produced in anaerobic fermentation is supplied to an absorption column sprinkled by water, from which the non-absorbed gases are fed to the first bio-reactor and/or the cogeneration unit and the aqueous solution from the absorption column is fed to a second bio-reactor.

5. The method of processing slops according to any of claims 1 to 4 wherein a portion of slops is added to photo-bioreactors.

6. The method of processing slops according to any of claims 1 to 5 wherein at least one photo-bioreactor is supplied with carbon dioxide from fermentation vessels used in production of bioalcohol.

7. The method of processing slops according to any of claims 1 to 6 wherein the first photo-reactor is operated as a closed photo-reactor.

8. The method of processing slops according to any of claims 1 to 7 wherein the second photo-reactor is operated as an open photo-reactor.
